# EUROPEAN PATENT APPLICATION

(11) **EP 3 208 768 A1**
(43) Date of publication of application: **23.08.2017**
(21) Application number: 15850451.4
(22) Date of filing: 14.10.2015
(51) Int. Cl.: G06Q 50/22, G06Q 50/24

(54) **CHARACTERISTIC EVALUATION DEVICE, CHARACTERISTIC EVALUATION SYSTEM, CHARACTERISTIC EVALUATION METHOD, AND CHARACTERISTIC EVALUATION PROGRAM**

(30) Priority: 15.10.2014 JP 2014210865; 24.08.2015 JP 2015165203
(71) Applicant: Omron Corporation, Kyoto-shi, Kyoto 600-8530 (JP); Kyushu University, National University Corporation, Fukuoka-shi, Fukuoka 812-8581 (JP)
(72) Inventor: KAN Eriko, Kyoto-shi Kyoto 600-8530 (JP); NAKAJIMA Hiroshi, Kyoto-shi Kyoto 600-8530 (JP); TSUCHIYA Naoki, Kyoto-shi Kyoto 600-8530 (JP); KOKUBO Ayako, Kyoto-shi Kyoto 600-8530 (JP); AZHAR Tazbirul, Daito-shi Osaka 574-0007 (JP); MASAKI Yoshinori, Fukuoka-shi Fukuoka 812-8581 (JP)
(74) Representative: Hinkelmann, Klaus
(86) International application number: PCT/JP2015/079088
(87) International publication number: WO 2016/060180

(57) **Abstract**

A characteristic evaluation apparatus (1) cumulatively stores, in a measurement history DB (13), measurement information (measurement date and time, measurement value and the like) that is obtained by measuring, by a weight scale (3) and the like, biological information or behavior information of a subject person. An arithmetic processing unit (11) evaluates psychological characteristics of the subject person with respect to a biological change or a behavioral change as a goal for the subject person based on a history of the measurement information of the subject person that is stored in the measurement history DB (13). A communication unit (14) outputs evaluation results evaluated by the arithmetic processing unit (11) with respect to the psychological characteristics of the subject person.

## Description

### Technical Field

The present invention relates to a characteristic evaluation apparatus configured to evaluate psychological characteristics of a subject person with respect to a biological change or a behavioral change as a goal for the subject person based on measurement information obtained by measuring biological information, behavior information and the like using various measurement instruments, and also relates to a characteristic evaluation system, a characteristic evaluation method and a characteristic evaluation program. Here, the biological change means a change in measurable biological information. The behavioral change means a change in a behavioral pattern that has become a habit.

### Background Art

These days, the number of people who try to lose weight for the purpose of preventing lifestyle related diseases such as diabetes and high blood pressure. On the other hand, some people try to gain weight for the purpose of improving low blood pressure or sensitivity to cold. Here, the increase and decrease of the weight are collectively referred to as weight management. In medical institutions, lifestyle modification menus concerning meals and exercises are proposed to the people who wish to manage the weight (hereinafter collectively referred to as a "subject person") so that they can reach their goal weight.

Also, as one of the information providing services using the internet, there is a service that provides the subject person with information on lifestyle modification by means of an e-mail or a website. For example, there is a service that conducts questionnaires to the subject person so as to supply information on lifestyle modification in accordance with the subject person's self-efficacy estimated based on answers to the questionnaires (see Patent Document 1).

The self-efficacy is a psychological term introduced by Albert Bandura, psychologist, which means conviction or perspective of how likely one is to successfully execute behavior required to obtain a certain outcome. The self-efficacy is classified into an efficacy expectation and an outcome expectation.

The efficacy expectation is an expectancy that one is to succeed at a task (here, modifying the lifestyle such as meals and exercises in order to lose weight). The outcome expectation is an expectancy that such a behavior is to lead to his/her desirable outcome (here, loss in weight).

For example, even when a weight loss menu associated with exercises or a weight loss menu associated with diet is suggested to the subject person for the purpose related to the biological change such as weight loss, the subject person is likely to fail to execute the menu if he/she has the low outcome expectation or the low efficacy expectation.

For this reason, in Patent Document 1, the questionnaire is conducted to the subject person so as to estimate the subject person's self-efficacy, so that a weight loss menu associated with exercises or a weight loss menu associated with diet is provided to the subject person as information on lifestyle modification according to the subject person's self-efficacy.

### Prior Art Document

### Patent Document

[Patent Document 1] JP 2001-022837 A

### Summary of the Invention

### Problem to be Solved by the Invention

However, the estimation of the self-efficacy based on the conduction of the questionnaire presupposes that the subject person answers the questionnaire. That is, it is necessary for the subject person to consume time and labor for answering the questionnaire. Also, when the questionnaires are repeatedly conducted in order to monitor the change in the subject person's self-efficacy, such questionnaires require impractical time and labor to answer. When the subject person should answer the repeatedly conducted questionnaires, he/she may be greatly affected by his/her psychological state at the respective times, although the questionnaire should be answered instinctively. In particular, if the questionnaire includes many questions, the subject person bothers with the answers, which may lead to biased answers or great variations in answers to the questionnaire. As a result, it is difficult to correctly estimate the subject person's self-efficacy.

An object of the present invention is to provide technology capable of easily evaluating psychological characteristics (for example, self-efficacy) of a subject person with respect to a biological change or a behavioral change as a goal for the subject person.

### Means for Solving the Problem

In order to achieve the above object, the characteristic evaluation apparatus of the present invention includes an input unit, an information storage unit and an evaluation unit, which are described later.

The input unit is to input: measurement information obtained by measuring biological information or behavior information, attribute information, or goal information of a subject person.

Here, examples of the biological information include, but not limiter to: a weight that can be measured by a weight scale; a weight, a body fat percentage, a visceral fat level, a basal metabolism, a skeletal muscle rate, a body age, and a BMI that can be measured by a weight and body composition meter; subcutaneous fat thickness that can be measured by an adipometer; a visceral fat level that can be measured by a visceral fat meter; a heart rate that can be measured by a heart rate monitor; systolic blood pressure and diastolic blood pressure that can be measured by a sphygmomanometer; and a body temperature that can be measured by a clinical thermometer. Examples of the behavior information include, but not limited to: the number of steps taken (which can be further divided into the number of steps taken when walking fast or the number of steps taken when mounting the steps), calorie consumption, and a fat combustion amount that can be measured by a pedometer or an activity monitor; the number of mastication that can be measured by a masticator; duration of jogging and the like that can be measured by a life log; sleeping hours that can be measured by a sleeping meter; and a moving distance that can be measured by a GPS. Examples of the measurement information include, but not limited to: measurement date and time; and a measurement value. Examples of the attribute information include, but not limited to: sex, age or generation of the subject person; a height that cannot be measured by the weight scale; and a BMI calculated by the above height and the measured weight. Examples of the goal information include, but not limited to: respective goal values of items such as the weight and the body composition; and a goal achievement period.

The information storage unit stores the measurement information, the attribute information, or the goal information input from the input unit. For example, such measurement information and the like may be stored on the cloud.

The information storage unit stores, for example, a history of the measurement date and time of the subject person's weight measured by the weight scale.

The evaluation unit evaluates psychological characteristics of the subject person with respect to a biological change or a behavioral change as a goal for the subject person based on the measurement information, the attribute information, or the goal information. For example, as the subject person has a higher self-efficacy with respect to the biological change (losing weight), he/she has a higher interest in his/her bodily changes. As a result, the subject person is likely to measure frequently the weight by the weight scale so as to confirm his/her current weight, which means a change in the measurement condition (the measurement frequency can be exemplarily presented, however, the number of measurement and the interval of measurement may also be used). That is, it is possible to estimate the subject person's self-efficacy with respect to the biological change (losing weight) based on the measurement condition (for example, measurement frequency) of the weight by the weight scale.

With the above-described configuration of the characteristic evaluation apparatus, it is possible to easily evaluate the psychological characteristics (for example, self-efficacy) of the subject person with respect to the biological change or the behavioral change as the goal for the subject person based on the measurement condition (for example, measurement frequency) of the living body. An analysis is performed in order to interpret the measurement information stored in the information storage unit. Thus, the psychological characteristics such as the self-efficacy are estimated based on features and patterns obtained by the analysis. The above processing is repeatedly performed every day, accordingly, the estimated psychological characteristics such as the self-efficacy are automatically updated while the past evaluation results are accumulated as case data. Also, since the questionnaire is not conducted to the subject person, it is not necessary for the subject person to consume time and labor. However, if necessary, the questionnaire to the subject person can be combined with the above processing, and furthermore, it is possible to automatically transmit, collect and evaluate such a questionnaire.

Also, in the characteristic evaluation apparatus of the present invention, the measurement information that is stored in the information storage unit may include at least a measurement date and time, and the evaluation unit may evaluate the psychological characteristics by means of evaluation points according to the measurement condition that is obtained from a history of the measurement information.

Also, in the characteristic evaluation apparatus of the present invention, the evaluation unit may change, in stages, a method for calculating the evaluation points calculated based on the measurement information when the measurement frequency included in the measurement condition, the measurement information, the attribute information, or the goal information are not less than respectively predetermined threshold values, or meet respectively predetermined conditions.

In the characteristic evaluation apparatus of the present invention, the evaluation unit may interpolate lack of the measurement information based on the behavior information.

The characteristic evaluation apparatus of the present invention may further include an output unit configured to output evaluation results evaluated by the evaluation unit with respect to the psychological characteristics of the subject person. The output unit may output, to the subject person, a message as the evaluation results according to the evaluation points evaluated by the evaluation unit with respect to the psychological characteristics of the subject person.

In the characteristic evaluation apparatus of the present invention, the measurement information stored in the information storage unit may include a measurement value associated with the measurement date and time when the biological information or the behavior information is measured. The output unit may output, to the subject person, a message as the evaluation results according to: the evaluation points evaluated by the evaluation unit with respect to the psychological characteristics of the subject person; and a change in the measurement value.

With the above-described configuration of the characteristic evaluation apparatus, it is possible to support the subject person who addresses the biological change or the behavioral change (for example, weight management).

Also, in a characteristic evaluation system of the present invention includes: a measurement instrument configured to measure the biological information or the behavior information of the subject person; and the characteristic evaluation apparatus according to any of the above description. The measurement information measured by the measurement instrument, the attribute information, or the goal information are directly or indirectly transmitted to the input unit of the characteristic evaluation apparatus.

Also, a characteristic evaluation method of the present invention includes: an information storage step of storing the measurement information obtained by measuring the biological information or the behavior information, the attribute information, or the goal information of the subject person; an evaluation step of evaluating the psychological characteristics of the subject person with respect to the biological change or the behavioral change as the goal for the subject person based on the measurement information, the attribute information, or the goal information of the subject person that are stored in the information storage step; and an output step of outputting evaluation results evaluated with respect to the psychological characteristics of the subject person in the evaluation step.

Also, a characteristic evaluation program that is configured to cause a computer to execute the characteristic evaluation method as described above is also within the scope of the present invention.

### Effect of the Invention

With the characteristic evaluation apparatus, the characteristic evaluation method and the characteristic evaluation program of the present invention, it is possible to easily evaluate psychological characteristics (for example, self-efficacy) of a subject person with respect to a biological change or a behavioral change as a goal for the subject person.

### Brief Description of the Drawings

[FIG. 1]

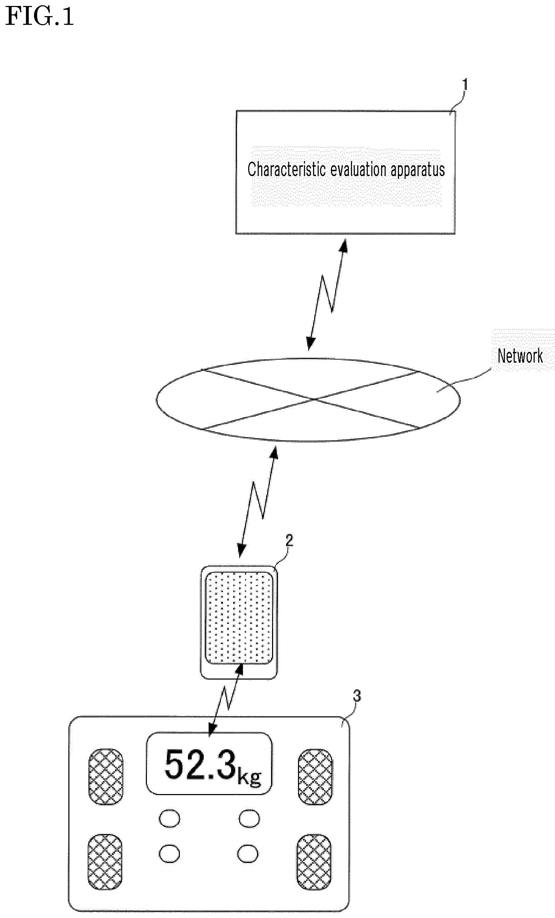

   FIG. 1 is a schematic diagram showing a configuration of a health support system according to a first embodiment.
[FIG. 2]

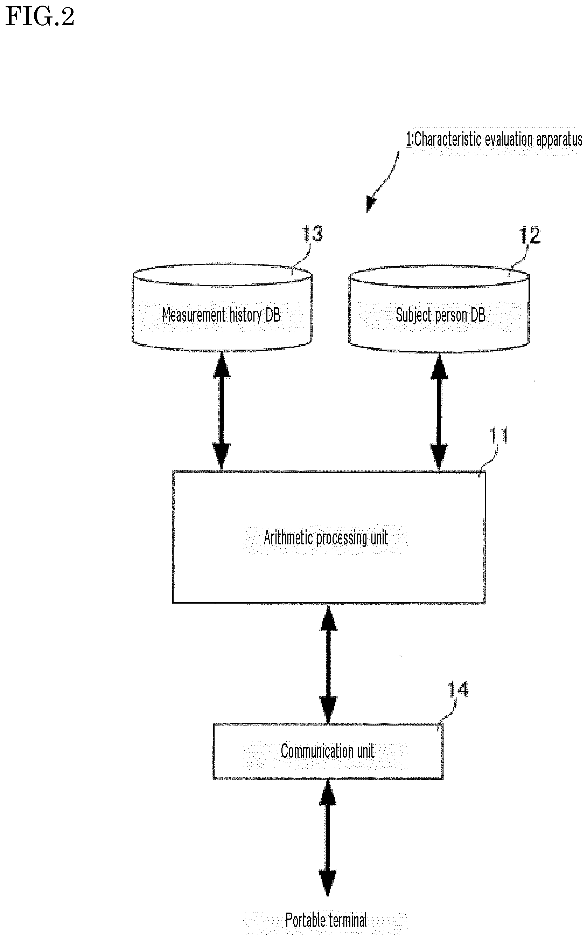

   FIG. 2 is a block diagram showing a configuration of a main part of a characteristic evaluation apparatus.
[FIG. 3]

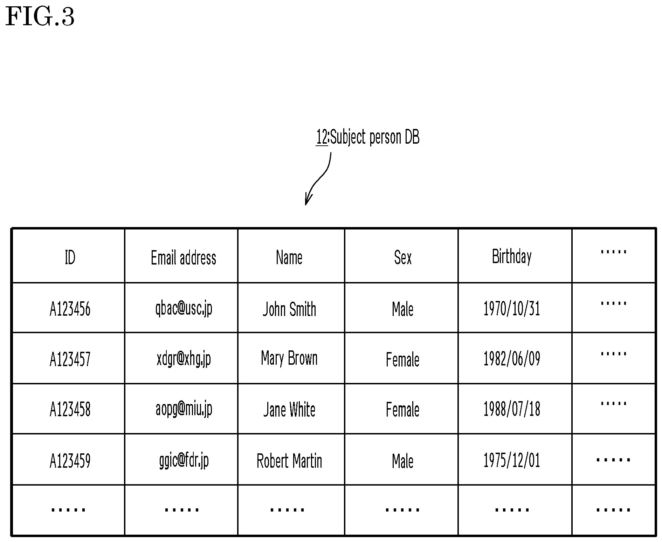

   FIG. 3 is a diagram showing a subject person database.
[FIG. 4]

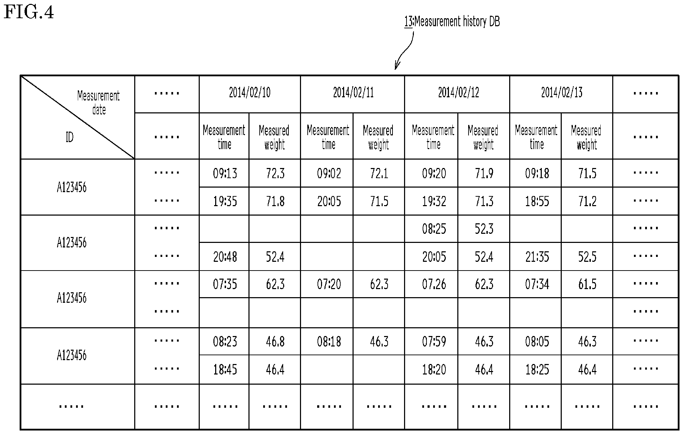

   FIG. 4 is a diagram showing a measurement history database.
[FIG. 5]

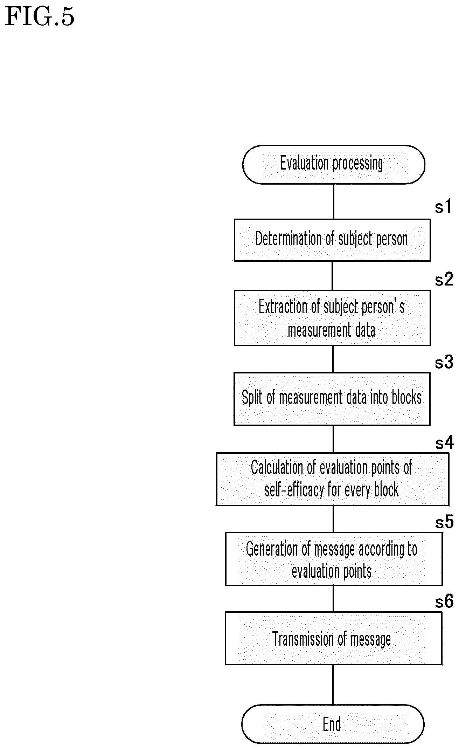

   FIG. 5 is a flowchart showing evaluation processing.
[FIG. 6]

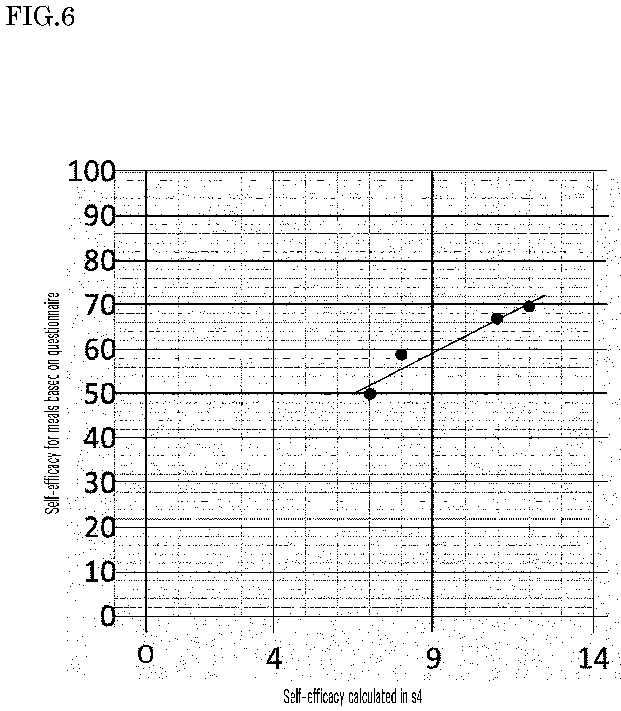

   FIG. 6 is a graph showing a correlation between self-efficacy for meals based on a questionnaire and self-efficacy calculated in s4.
[FIG. 7]

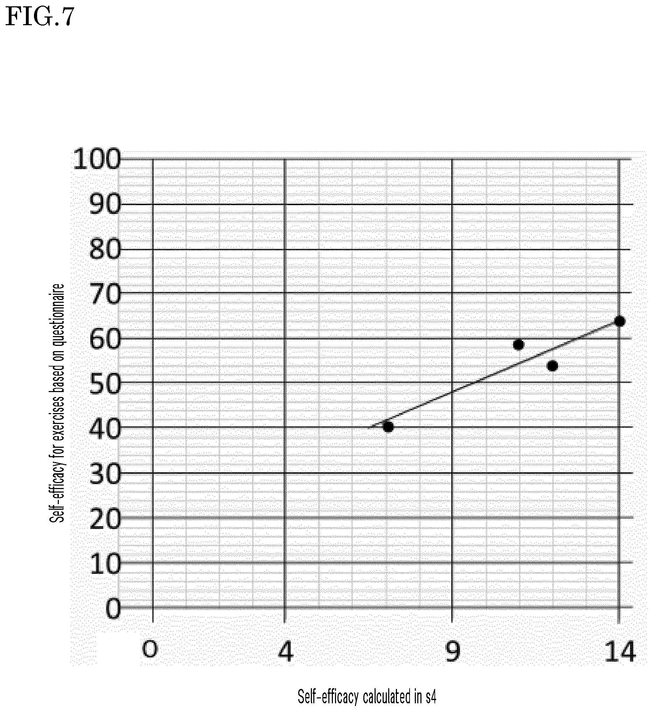

   FIG. 7 is a graph showing a correlation between self-efficacy for exercises based on a questionnaire and self-efficacy calculated in s4.
[FIG. 8]

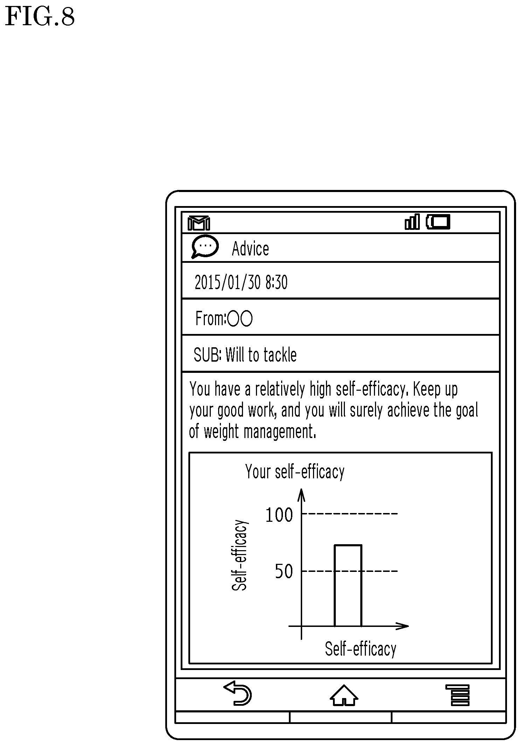

   FIG. 8 is a diagram exemplarily showing a case in which evaluation results including evaluation points are supplied to the subject person.
[FIGS. 9]

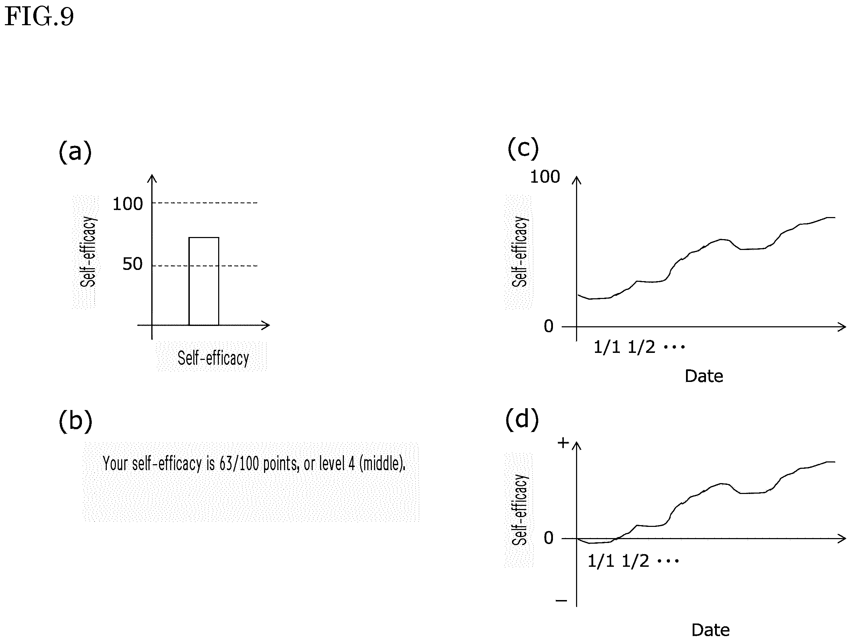

   FIGS. 9(a) to 9(d) are diagrams showing examples to indicate the evaluation points.
[FIGS. 10]

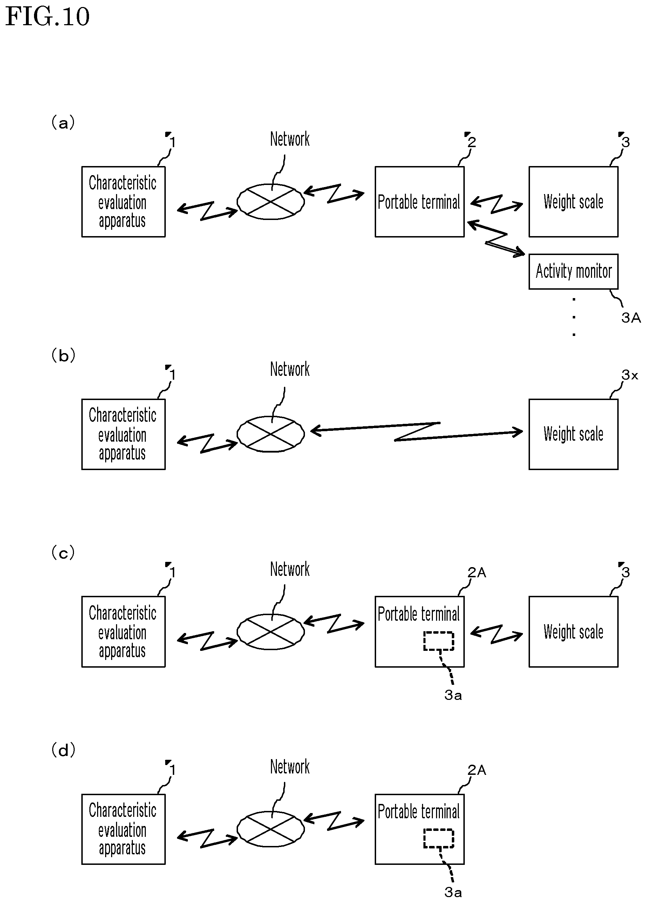

   FIGS. 10(a) to 10(d) are schematic diagrams showing respective configurations of the health support system according to various variations of the first embodiment.
[FIG. 11]

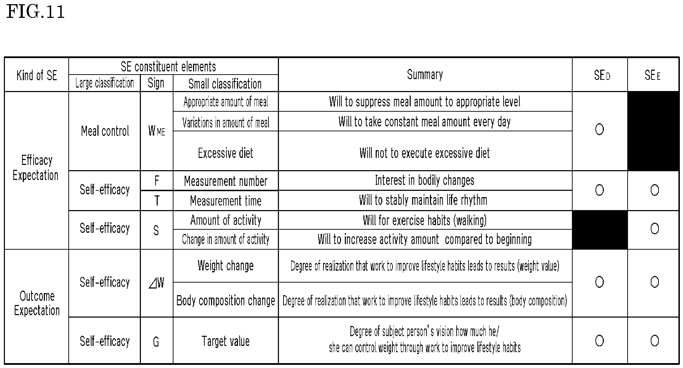

   FIG. 11 is a table showing specific examples of elements constituting a meal SE_{D} and an exercises SEE and their correspondence relation in a second embodiment.
[FIG. 12]

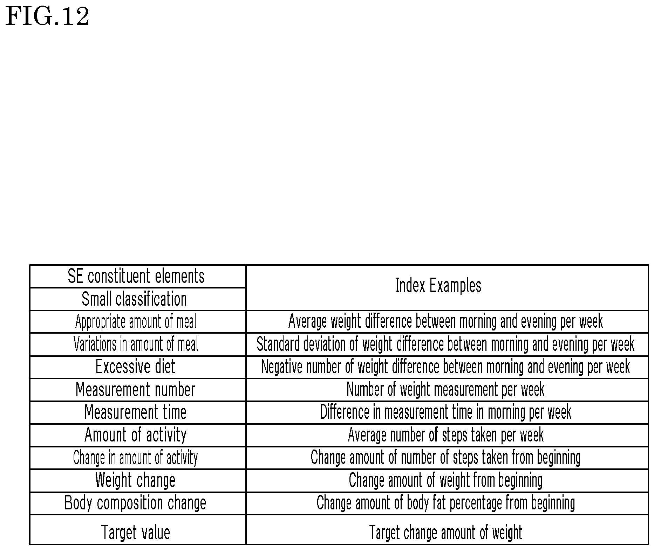

   FIG. 12 is a table showing index examples corresponding to a small classification of the constituent elements in every kinds of SE that are the same as those in FIG. 11.
[FIG. 13]

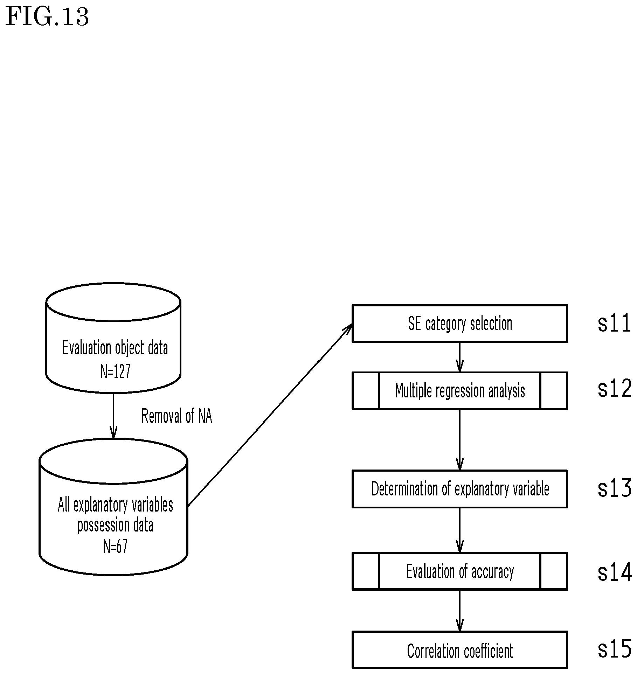

   FIG. 13 is a flowchart exemplarily showing a method for formulating respective estimation expressions for the meal SE_{D} and the exercise SE_{E} by the multiple regression analysis.
[FIGS. 14]

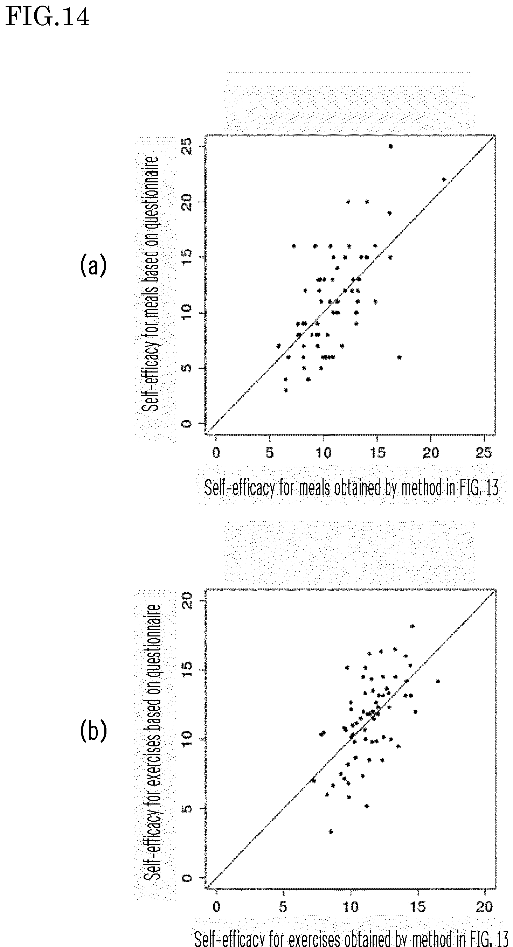

   FIGS. 14(a) and 14(b) are graphs respectively showing correlations between the meal SE_{D}/exercise SEE obtained by the method indicated in FIG. 13 and the self-efficacy based on the questionnaire.
[FIG. 15]

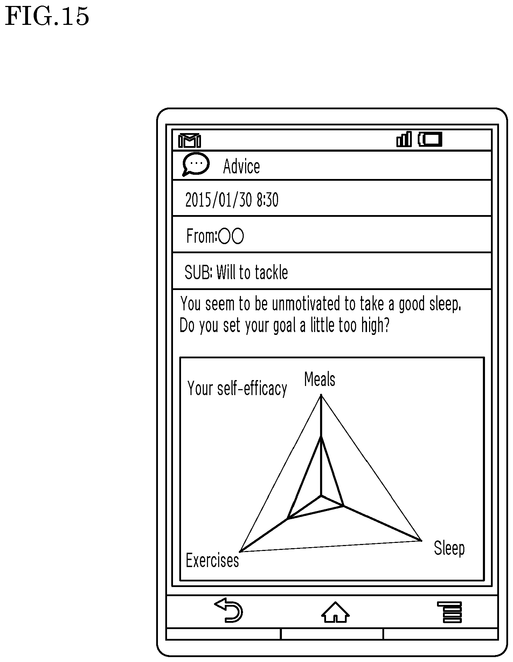

   FIG. 15 is a diagram exemplarily showing a case in which the evaluation results including the evaluation points are supplied.
[FIGS. 16]

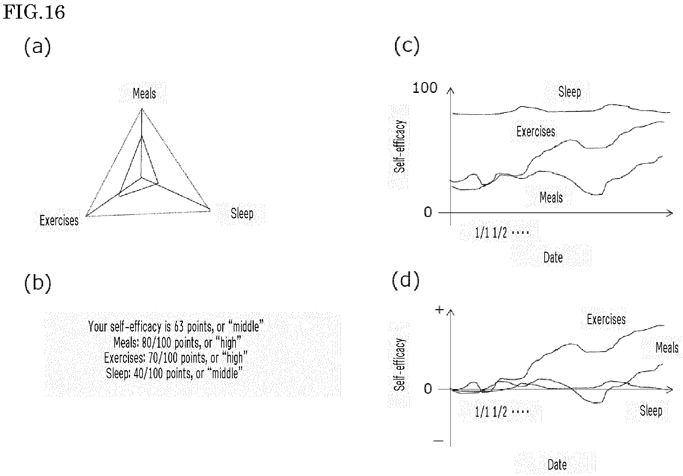

   FIGS. 16(a) to 16(d) are diagrams showing examples to indicate the evaluation points.
[FIG. 17]

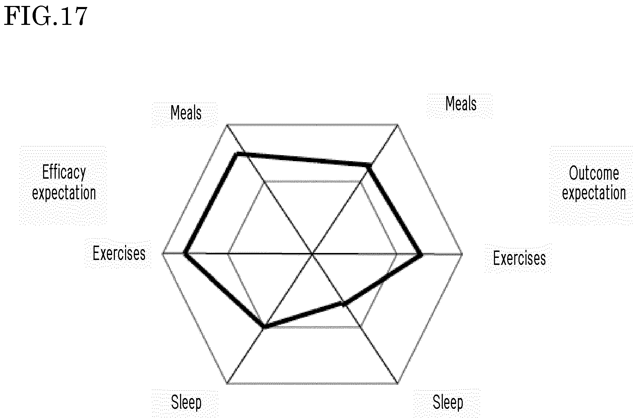

   FIG. 17 is a diagram showing an example to indicate the evaluation points in a manner of a radar chart.

### Modes for Carrying Out the Invention

Hereinafter, several embodiments of the present invention will be described with reference to the drawings.

### <First Embodiment>

### 1.1 Configuration of Health Support System According to First Embodiment

FIG. 1 is a schematic diagram showing a configuration of a health support system according to the first embodiment of the present invention. The health support system according to the first embodiment is to support a subject person who addresses the weight management to lose weight. For example, a weight loss menu associated with diet or a weight loss menu associated with exercises is presented to the subject person in order to support the subject person. The health support system includes a characteristic evaluation apparatus 1, a portable terminal 2 and a weight scale 3. The subject person who addresses the weight management possesses the portable terminal 2 and the weight scale 3. In this example, the portable terminal 2 is paired with the weight scale 3.

In FIG. 1, only a pair of portable terminal 2 and weight scale 3 possessed by one subject person is exemplarily shown, however, FIG. 1 does not mean that only his/her portable terminal 2 is connected to the characteristic evaluation apparatus 1 through the network. Many portable terminals 2 of the respective subject persons are connected to the characteristic evaluation apparatus 1 through the network.

The weight scale 3 is capable of measuring the weight and has a short-distance wireless communication function for transmitting the measurement result to the portable terminal 2 through the short-distance wireless communication. Examples of the short-distance wireless communication function include, but not limited to, communications through the NFC and the FeliCa (registered trademark), and a communication through the Bluetooth (registered trademark). The weight scale 3 may be a weight and body composition meter (or simply referred to as a body composition meter) that can measure not only the weight but also the body fat percentage and the body mass index (BMI). This type of weight and body composition meter has been already commercially available (for example, see http://www.healthcare.omron.co.jp/sp/hbf252f/). It is also possible to use another measurement instrument capable of measuring of biological information of the subject person.

The portable terminal 2 has the short-distance wireless communication function to perform the short-distance wireless communication with the weight scale 3 and the network communication function to perform communication with the characteristic evaluation apparatus 1 through the network such as the internet. This type of portable terminal 2 has been already commercially available as a smartphone, however, it is also possible to use a tablet computer or a wearable computer. Furthermore, a personal computer and the like can be used in place of the portable terminal 2. However, alternative apparatuses of the portable terminal 2 are not limited to the above.

FIG. 2 is a block diagram showing a configuration of a main part of the characteristic evaluation apparatus according to the first embodiment. The characteristic evaluation apparatus 1 includes: an arithmetic processing unit 11; a subject person database 12 (hereinafter referred to as the subject person DB 12); a measurement history database 13 (hereinafter referred to as the measurement history DB 13); and a communication unit 14.

The arithmetic processing unit 11 controls operations of a main body of the characteristic evaluation apparatus 1, and evaluates psychological characteristics (here, self-efficacy) of the subject person with respect to a weight management (here, losing weight) that is a goal biological change for the subject person. Also, the arithmetic processing unit 11 determines a message to the subject person according to evaluation points of the evaluated psychological characteristics. The processing performed by the arithmetic processing unit 11 is described later in detail. The arithmetic processing unit 11 has a computer in which a characteristic evaluation program according to the present invention is installed. The computer executes a characteristic evaluation method according to the present invention. Also, the arithmetic processing unit 11 has a configuration corresponding to an evaluation unit of the present invention.

The subject person DB 12 is a database to manage the subject person. FIG. 3 is a diagram showing the subject person database. Here, the subject person means members who subscribe to the health support system and address the weight management. The subject person DB 12 is a database in which are registered respective subject person records associating, for each subject person, the ID for identifying the subject person (identification code), the subject person's registered email address, the subject person's name, the subject person's sex, the subject person's birthday and the like.

FIG. 4 is a diagram showing the measurement history database. The measurement history DB 13 stores, for each subject person, measurement data associating the weight measurement date and time and the measured weight. The measurement history DB 13 has a region to store, for each subject person, the measurement data measured twice a day (in the morning and in the evening). In FIG. 4, the region where no measurement data is stored means that there is no measurement data because the subject person has not measured the weight. The measurement history DB 13 corresponds to an information storage unit of the present invention. As shown in FIG. 4, the time series pattern of the weight measurement differs for each subject person.

Here, the measurement history DB 13 has the region to store, for each subject person, the measurement data measured twice a day (in the morning and in the evening). However, the measurement history DB 13 may have a region to store the measurement data measured three times or more a day. Alternatively, the measurement history DB 13 does not necessarily limit in advance the region to store the measurement data for one day, but stores sequentially the data associating the measurement date and time and the measurement value. Also, it is possible to set previously the getting-up time slot and the going-to-bed time slot so as to be adapted to the subject person's lifestyle, and the measurement data may be stored according to the respective time slots. In this case, the measurement executed at the time other than the set time slots may be omitted, or may be treated as the measurement data in the other time slot.

The communication unit 14 communicates, through the network, with the portable terminal 2. In this way, the weight of the subject person that is measured by the weight scale 3 as the outside measurement instrument is input from the communication unit 14, along with the measurement date and time, via the portable terminal 2. Also, as described later, the message to the subject person that is generated by the arithmetic processing unit 11 is output to the portable terminal 2. That is, the communication unit 14 corresponds to an input unit and an output unit of the present invention.

### 1.2 Operations of Health Support System According to First Embodiment

Hereinafter, operations of the health support system according to the first embodiment is described.

In the health support system, one who wants to be a subject person (i.e., candidate) subscribes to the health support system. For example, the candidate inputs required items such as name, sex, birthday, aim (losing/increasing weight etc.), email address and the like in a registration page published on the internet by the characteristic evaluation apparatus 1. An ID is given to the candidate based on the contents that he/she has input in the registration page, and the candidate is registered in the subject person DB 12. Thus, the candidate can be the subject person upon completion of the registration to the health support system. Also, the characteristic evaluation apparatus 1 notifies the newly registered subject person of the ID given to him/her.

Next, the weight measurement by the subject person is described. When the subject person measures the weight, the weight scale 3 temporarily stores the measurement data associating the measurement date and time and the measured weight in a memory and the like provided in the main body of the weight scale 3. The weight scale 3 transmits the measurement data temporarily stored in the memory to the portable terminal 2 through the short-distance wireless communication.

The weight scale 3 and the portable terminal 2 may communicate through a wire line network. Also, the subject person may input the measurement date and time and the weight measured by the weight scale 3 to the portable terminal 2.

The portable terminal 2 gives an ID to the measurement data transmitted from the weight scale 3, and transmits the measurement data with ID to the characteristic evaluation apparatus 1. When the characteristic evaluation apparatus 1 receives, at the communication unit 14, the measurement data with ID transmitted from the portable terminal 2, the characteristic evaluation apparatus 1 stores the measurement data in the measurement history DB 13. Thus, the characteristic evaluation apparatus 1 stores the measurement data for each subject person in the measurement history DB 13. As described above, the measurement data includes not only the measurement value but also the measurement date and time.

Next, evaluation processing in the arithmetic processing unit 11 is described. The evaluation processing is to evaluate psychological characteristics (here, self-efficacy) of the subject person with respect to the weight management that is a goal biological change for the subject person. As described above, the self-efficacy is a psychological term introduced by Albert Bandura, psychologist, which means conviction or perspective of how likely one is to successfully execute behavior required to obtain a certain outcome. The evaluation processing is processing to quantitatively evaluate the deepness (strength) of the subject person's conviction of how he/she is to succeed at execution of the weight loss menu associated with diet or the weight loss menu associated with exercises that is presented to the subject person. Also, in the evaluation processing, the message to the subject person is generated according to the evaluated self-efficacy SE so as to input the message from the communication unit 14. The message to the subject person is evaluation results.

FIG. 5 is a flowchart showing the evaluation processing. The arithmetic processing unit 11 determines the subject person who is subjected to the evaluation processing at this time (s1). In s1, any subject person whose ID is registered in the subject person DB 12 is determined as the subject person to be subjected to the evaluation processing at this time.

The arithmetic processing unit 11 extracts the measurement data that is stored in the measurement history DB 13 regarding the subject person determined in s1 (s2).

The arithmetic processing unit 11 splits the measurement data extracted in s2 into blocks divided according to a predetermined period (in this example, one week) (s3). In s3, the subject person's measurement data for one week from Monday to next Sunday is distributed in one block.

The arithmetic processing unit 11 calculates the evaluation points of the self-efficacy SE for every block divided in s3 (s4). Here, the self-efficacy SE is expressed by the following expression, as the function of a measurement frequency F:$SE = f \left(F\right) .$

In s4, in each day of the block for calculating the evaluation points of the self-efficacy SE, if the weight is measured by the weight scale 3 in the morning time slot, "1 point" is added. Also, if the weight is measured by the weight scale 3 in the evening time slot, "1 point" is added. That is, the maximum value of the evaluation points of the self-efficacy SE calculated for every block is "14 points" when the weight is measured at least once for every morning time slot and every evening time slot of the days that belong to each block. Also, the minimum value is "0 point" when the weight is not at all measured by the weight scale 3 throughout the days belonging to the block. Thus, in s4, the self-efficacy SE of the subject person is evaluated in 15 stages from 0 point to 14 points.

Also, as described above, the subject person's measurement data for one week from Monday to next Sunday is distributed in one block so as to calculate the evaluation points of the self-efficacy SE for every block. Thus, it is possible to obtain not only the current evaluation points of the self-efficacy SE of the subject person but also the temporal change of the evaluation points of the self-efficacy SE of the subject person.

Here, reliability of the self-efficacy SE calculated in s4 is described.

For example, the self-efficacy for meals is a self-efficacy with respect to meals that is confidence for not eating too much when being in the situation that one tends to eat too much. As a scale of this self-efficacy, a normalization of the questionnaire elaborated by Dr. Matthew M. Clark et al., in Mayo Clinic based in Minnesota, the United States of America is known. It is normalized so that the total points of answers has 100 points as the full marks. The questionnaire is constituted by 20 questions from Q1 to Q20 as shown below. This is a questionnaire for evaluating the self-efficacy for meals by answering to each question (Q1 to Q20) on a scale of "0 point (Min)" (not at all confident) to "9 points (Max)" (very confident), i.e., in 10 stages.
Q1: I can resist eating when I am anxious or irritated.
Q2: I can control my eating on the weekends (holidays).
Q3: I can refrain from eating even saying "no" to the people around.
Q4: I can resist eating when I feel run-down.
Q5: I can resist eating when I am watching TV.
Q6: I can resist eating when I am depressed.
Q7: I can resist eating even when various sorts of foods are available.
Q8: I can decline a second helping.
Q9: I can resist eating when I have a headache.
Q10: I can resist eating when I am reading a book.
Q11: I can resist eating when I am in anger.
Q12: I can resist eating when I am on a banquet or a drinking party.
Q13: I can refuse to eat when I am encouraged to eat by the people around.
Q14: I can resist eating when I have a pain.
Q15: I can resist eating before going to bed.
Q16: I can resist eating when I fell down.
Q17: I can resist eating even when high-calorie foods are available.
Q18: I can refuse to eat in spite of opposition of the people around.
Q19: I can resist eating when I feel sick.
Q20: I can resist eating when I feel delightful.

The above questions Q1 to Q20 are cited from Clark. "Self-efficacy in weight management." Journal of Consulting and Clinical Psychology, Vol 59(5), Oct 1991, pp. 739-744.

FIG. 6 is a graph showing a correlation between the self-efficacy for meals based on the above questionnaire and the self-efficacy SE calculated in s4. In FIG. 6, the vertical axis indicates the score of the self-efficacy for meals that is evaluated based on the above questionnaire, with the full marks of which being set to 100 points. The horizontal axis indicates the evaluation points (evaluation points calculated in s4) based on the frequency of weight measurement by the weight scale 3. There is a high correlation between the self-efficacy for meals based on the above questionnaire and the self-efficacy SE calculated in s4 (R² equals 0.9 or more). Thus, it is estimated that the evaluation points of the self-efficacy SE calculated in s4 is sufficiently reliable relative to the self-efficacy for meals.

On the other hand, the self-efficacy for exercises is a self-efficacy with respect to exercises that is confidence for continuing physical activities, taking time for exercising, or exercising under the condition that prevents the exercise. As a scale of this self-efficacy, a normalization of the questionnaire elaborated by Takashi Muto et al. at Dokkyo Medical University is known. It is normalized so that the total points of answers has 100 points as the full marks. The questionnaire is constituted by 13 questions from Q31 to Q43 as shown below. This is a questionnaire for evaluating the self-efficacy for exercises by answering to each question (Q31 to Q43) with 3 options, i.e., "Yes (3 points)", "No (1 point)", and "Neither (2 points)".
Q31: I can exercise even after a long, tiring day at work.
Q32: I can set aside time for exercise when I am busy.
Q33: I can exercise when I have household chores to attend to.
Q34: I can exercise when I have excessive demands at work.
Q35: I can exercise when I am in a bad condition.
Q36: I can exercise even though I am depressed.
Q37: I can exercise when the weather is not good.
Q38: I can exercise when I don't have a trainer.
Q39: I can exercise when I don't have company.
Q40: I can exercise even when I feel lack of skills.
Q41: I can exercise when facilities or equipment is poor.
Q42: I can exercise independent of athletic meetings.
Q43: I can get up early to exercise.

The above questions Q31 to Q43 are cited from Muto. T, "The development of a self-efficacy scale for exercise behavior." The Keio Journal of Medicine 1992, 41(1), pp. 21-24.

FIG. 7 is a graph showing a correlation between the self-efficacy for exercises based on the above questionnaire and the self-efficacy SE calculated in s4. In FIG. 7, the vertical axis indicates the score of the self-efficacy for exercises that is evaluated based on the above questionnaire, with the full marks of which being set to 100 points. The horizontal axis indicates the evaluation points (evaluation points calculated in s4) based on the frequency of weight measurement by the weight scale 3. There is a high correlation between the self-efficacy for exercises based on the above questionnaire and the self-efficacy SE calculated in s4 (R² equals 0.8 or more). Thus, it is estimated that the evaluation points of the self-efficacy SE calculated in s4 is also sufficiently reliable relative to the self-efficacy for exercises.

In other words, it is estimated that the evaluation points of the self-efficacy SE calculated in s4 is sufficiently reliable relative to both the self-efficacy for meals and the self-efficacy for exercises, which are associated with the weight management.

The arithmetic processing unit 11 generates the message to the subject person according to the evaluation points of the self-efficacy SE calculated in s4 (s5). In s5, for example, the arithmetic processing unit 11 determines the latest evaluation points of the self-efficacy SE of the subject person in three stages, i.e., "low (0 to 4 points)", "middle (5 to 9 points)" and "high (10 to 14 points)".

Then, the arithmetic processing unit 11 generates a message as the evaluation results, for the subject person whose self-efficacy SE has been evaluated as "low", in order to notify that his/her self-efficacy related to the weight management is low and to encourage the subject person to increase the self-efficacy so as to achieve the goal of weight management. For example, the message "your motivation to go on the weight management seems to be low. Continue your weight management with a strong will." is generated as the evaluation results so as to encourage the subject person to positively go on the weight management.

The arithmetic processing unit 11 generates a message, for the subject person whose self-efficacy SE has been evaluated as "middle" that means a general self-efficacy related to the weight management, in order to further increase the subject person's self-efficacy. For example, the message "Keep trying a little more, and you are highly likely to achieve the goal of weight management." is generated as the evaluation results so as to encourage the subject person to successfully go on the weight management.

The arithmetic processing unit 11 generates a message, for the subject person whose self-efficacy SE has been evaluated as "high", in order to keep the subject person's self-efficacy for decreasing the weight. For example, the message "Keep up your good work, and you will surely achieve the goal of weight management." is generated as the evaluation results so as to praise the subject person.

In the characteristic evaluation apparatus 1, the message generated by the arithmetic processing unit 11 in s5 is transmitted to the subject person via email by the communication unit 14 (s6). The subject person receives the email by his/her portable terminal 2, personal computer and the like.

As described above, with the characteristic evaluation apparatus 1 according to the first embodiment, it is possible to easily and accurately evaluate the subject person's self-efficacy with respect to the biological change (here, weight management) as a goal for the subject person. Also, since the questionnaires are not conducted to the subject person, it is not necessary for the subject person to consume time and labor. Furthermore, since the characteristic evaluation apparatus 1 generates the message to be transmitted to the subject person according to the evaluation points calculated in s4, it is possible to support the subject person so that he/she can achieve the goal of the weight management.

Also, in s6, not only the message generated in s5 but also the evaluation points of the self-efficacy SE calculated in s4 with respect to the weight management may be transmitted to the subject person as shown in FIG. 8. Alternatively, the evaluation results including the evaluation points may be provided to an instructor. Thus, it is possible to improve instruction efficiency or to lead to a better support.

As an example of presentation of the evaluation points, it is possible to use a bar graph as shown in FIGS. 8 and 9(a). In this way, it is easy to recognize the strength of the self-efficacy SE. If the evaluation points are displayed as the values or levels as shown in FIG. 9(b), it is easy to have an improvement desire. If the time series variation is displayed as a graph as shown in FIG. 9(c), it is easy to recognize the change in the self-efficacy SE. Furthermore, if the time series variation is displayed as a graph with a start time as a reference as shown in FIG. 9(d), it is easy to recognize the change from the start time. However, the display manner of the evaluation points is not limited to the above examples.

In the above description, the message to the subject person is generated according to the evaluation points of the subject person's latest self-efficacy SE. However, it is also possible to generate the message to the subject person according to, in addition to the evaluation points, the temporal change of the evaluation points of the subject person's self-efficacy SE. For example, when the subject person's self-efficacy SE with respect to the weight management has a tendency to decrease (i.e., the evaluation points calculated in s4 has a tendency to decrease), it is possible to estimate that the subject person's motivation for weight management is decreased. Thus, the message such as "Maintain your weight management as before." is generated as the evaluation results. Also, when the subject person's self-efficacy SE with respect to the weight management has a tendency to increase (i.e., the evaluation points calculated in s4 has a tendency to increase), it is possible to estimate that the subject person's motivation for weight management is increased. Thus, the message such as "Keep up your good work of weight management." is generated as the evaluation results.

Also, it is possible to generate the message according to, in addition to the above, the variation in the measurement value of the weight. For example, to the subject person whose weight is on the increase, the message such as "Make a little more effort to continue your weight management." is generated as the evaluation results. On the other hand, to the subject person whose weight is on the decrease, the message such as "You can see the good results of the weight management. Keep up your good work." is generated as the evaluation results.

Furthermore, for the subject person whose self-efficacy SE with respect to the weight management is low, a diet menu or an exercise menu to be presented may be changed to a menu easier to perform even if the effect for losing weight is reduced. In this way, it is possible to gradually increase the subject person's self-efficacy SE with respect to the weight management. On the other hand, for the subject person whose self-efficacy SE with respect to the weight management is high, a diet menu or an exercise menu to be presented may be changed to a menu that can expect a greater effect for losing weight.

In the present invention, it is possible to perform the function of the evaluation processing shown in FIG. 5 in a manner in which the function is installed in the weight scale 3. In this case, the evaluation results may be displayed on the display part provided on the main body of the weight scale 3. The subject person is a person who measures the weight by the weight scale 3.

Also, the present invention is not limited to be applied to the weight management for losing weight as described above. It is possible to easily evaluate psychological characteristics (for example, self-efficacy) of the subject person based on the biological measurement frequency of the subject person even when the goal biological change of the subject person in order to prevent lifestyle related diseases is such as weight management for increasing weight, suppression of the body fat percentage, suppression of the body mass index (BMI) or suppression of blood pressure.

When the self-efficacy SE is "high", the evaluation may be more detailed using the evaluation expression based on "strength of the will to stably maintain the life rhythm" obtained from measurement time T in addition to "high interest in the bodily changes" obtained from the measurement frequency F.$SE = f \left(F, T\right) .$

For example, if the subject person works weekdays and has the weekends off, his/her measurement time (getting-up time/going-to-bed time) is likely to be different between the weekdays and the weekends. When the subject person performs measurement at the almost same time regardless of whether it is a weekday or a weekend, his/her lifestyle habits are stable, which results in a high self-efficacy SE with respect to leading a well-regulated life.

First, if the measurement of the weight is performed by the weight scale 3 in the morning and in the evening of each day for every block (for example, one week), the points according to the above are added and totaled so as to calculate the evaluation points. When the block is set to one week, the evaluation points for every block is in the range of from 0 to 14 points.

In the above description, the subject person's self-efficacy SE is determined in three stages, i.e., "low", "middle" and "high" based on the evaluation points of only the latest block. Here, the subject person's self-efficacy SE is determined based on the evaluation points of every block of the past fixed period including the latest block (for example, the past 4 weeks).

For example, if any one of the evaluation points of every block of the past fixed period is 6 points or less, it can be estimated that the measurement of the weight at least once a day has not yet been established as a habit. Thus, the subject person's self-efficacy SE is determined as "low". In contrast, all of the evaluation points of the respective blocks of the past fixed period are 10 points or more, it can be estimated that not only the measurement of the weight once a day but also the measurements in the morning and in the evening have been established as a habit to the some extent. Thus, the subject person's self-efficacy SE is determined as "high". If the evaluation points fall into neither of the above cases, the subject person's self-efficacy SE is determined as "middle". However, the manner of determination is not limited to the above. For example, the determination may be performed further in consideration of recorded status of the measurement data by an activity monitor (i.e., the average, the variation, the in-week fluctuation and the like of the number of steps taken and the calorie consumption).

Also, examples of the messages output according to the determination results include those described in the paragraphs below, but are not limited thereto. For example, it is possible to add the message according to the recorded status of the measurement data by an activity monitor 3A similarly to the determination processing as described above.

Self-efficacy SE: "low"
"Do you tend to forget the measurement of the weight? If so, how about thinking about relocation of the weight scale, for example?"
Self-efficacy SE: "middle"
"You can see the amount of meals and the amount of exercises (activities) of the day from the difference between the weight of the morning and the weight of the evening.

How about confirming this by measuring the weight continuously in the morning and in the evening?"
Self-efficacy SE: "high"
"You are working hard at measuring every day! Keep it up!"

### <Variation of First Embodiment>

The above-described health support system according to the first embodiment includes the characteristic evaluation apparatus 1, the portable terminal 2 and the weight scale 3 as shown in FIG. 1. However, the present invention is not limited thereto. FIGS. 10(a) to 10(d) are schematic diagrams showing respective configurations of the health support system according to various variations of the first embodiment of the present invention.

FIG. 10(a) is a diagram showing a configuration in which the activity monitor 3A is added to the health support system according to the first embodiment. The activity monitor 3A is capable of measuring the amount of activity (for example, the number of steps taken, the calorie consumption, and changes over time thereof) as behavior information of the subject person. The activity monitor 3A has, similarly to the weight scale 3, a short-distance wireless communication function for transmitting the measurement result to the portable terminal 2 through the short-distance wireless communication. Furthermore, it is possible to add other measurement instruments capable of measuring the biological change or the behavioral change of the subject person. With this configuration, it is possible to monitor more accurately the biological change or the behavioral change of the subject person, which results in improvement of the accuracy in evaluation of the psychological characteristics.

FIG. 10(b) is a diagram showing a configuration in which: the portable terminal 2 is omitted from the health support system according to the first embodiment; and the weight scale 3 is modified to a weight scale 3x by adding, to the weight scale 3, the network communication function capable of directly communicating with the characteristic evaluation apparatus 1. With this configuration, even when the subject person does not have the portable terminal 2, he/she can use the health support system. Similarly to FIG. 10(a), it is possible to add other measurement instruments capable of measuring the biological change or the behavioral change of the subject person (provided that such instruments have the network communication function capable of directly communicating with the characteristic evaluation apparatus 1).

FIG. 10(c) is a diagram showing a configuration in which: the activity monitor 3A is omitted from the health support system shown in FIG. 10(a); and the portable terminal 2 is modified to a portable terminal 2A by installing, in the portable terminal 2, activity monitor functions 3a that are almost the same as the activity monitor 3A. With this configuration, the subject person can just carry always the portable terminal 2 that is originally almost necessary to live a daily life, and is not required to carry the activity monitor 3A separated from the portable terminal 2. Thus, it is possible to reduce the subject person's burden in carrying always the activity monitor 3A in addition to the portable terminal 2. As a result, it is possible to prevent lack of the measurement data and the like caused by forgetting to carry the activity monitor 3A. The activity monitor functions 3a to be installed in the portable terminal 2 may be partially simplified compared to the various functions of the individual activity monitor 3A.

FIG. 10(d) is a diagram showing a configuration in which the weight scale 3 is omitted from the health support system shown in FIG. 10(c). With this configuration, the subject person can use the health support system without using the weight scale 3. In this case, it is possible, mainly, to evaluate the psychological characteristics of the subject person based on his/her behavioral change.

### <Second Embodiment>

As one variation of the first embodiment, in the second embodiment, the health support system shown in FIG. 10(a) is modified by increasing the measurement data and other data to be transmitted from the portable terminal 2 to the characteristic evaluation apparatus 1 and by improving: calculation processing of the evaluation points of the self-efficacy SE in the arithmetic processing unit 11 of the characteristic evaluation apparatus 1 (see s4 in FIG. 5); and message generation processing according to the evaluation points (see s5 in FIG. 5). Hereinafter, the difference of the second embodiment from the first embodiment is mainly described.

### 2.1 Data to be Transmitted from Portable Terminal 2 to Characteristic Evaluation Apparatus 1

In the health support system as shown in FIG. 10(a), the subject person's biological information and behavior information, which are the weight measured by the weight scale 3 and its measurement date and time as well as the amount of activity measured by the activity monitor 3A, are simply transmitted to the characteristic evaluation apparatus 1 via the portable terminal 2. In the meantime, if a position information detection function such as a GPS is installed in the portable terminal 2, it is possible to estimate, to some extent, activities of the subject person (for example, moving history, moving distance and moving speed) based on the measurement record. Furthermore, when referring to geographic information and the like based on the position information included in the moving history, it may be possible to grasp the kinds of the subject person's activities. If the subject person's schedule data is stored in the portable terminal 2, it may also give clue to the kinds of the subject person's activities. In addition, the data as described below will be beneficial information if it is registered in the portable terminal 2 or the weight scale 3: the subject person's attribute data (sex, age or generation, height that cannot be measured by the weight scale 3, BMI that is calculated based on the height and the measured weight, and the like); the goal data (respective goal values for every item such as the weight and the body composition, the period for achieving the goal value, and the like); and the experience on the weight loss program.

Thus, in the second embodiment, the portable terminal 2 transmits the subject person's behavior information and other pieces of information stored in the portable terminal 2 to the characteristic evaluation apparatus 1.

### 2.2 Calculation of Evaluation Points of Subject person's Self-efficacy and Generation of Message According to Evaluation Points

The self-efficacy SE with respect to the goal biological change of the subject person is related to a plurality of lifestyle habits such as meal habits, exercise habits, sleeping habits and the like. Thus, it is necessary to behaviorally change each lifestyle habit in order to achieve the goal biological change. For example, if the subject person has a low self-efficacy (mainly, efficacy expectation "SE efficacy") with respect to the weight loss menu associated with exercises and diet, he/she flinches from the menu feeling that he/she cannot do it, and consequently does not try the presented weight loss menu. Also, even when the subject person has a high efficacy expectation "SE efficacy", if the outcome expectation "SE outcome" is low, the subject person's motivation cannot be raised sufficiently, which may result in avoidance of the menu. On the other hand, as to the subject person who is determined to have a high self-efficacy SE as described in the first embodiment, the measurement number and frequency are high, which means that the difference between the weight of the morning and the weight of the evening both measured by the weight scale 3 can be recognized almost every day and that the measurement data by the activity monitor 3A is probably complete. Accordingly, it is possible to evaluate the self-efficacy SE in more detail. Thus, the evaluation stage may be shifted so as to calculate, with respect to the various lifestyle habits, the self-efficacy SE and furthermore the outcome expectation "SE outcome" and the efficacy expectation "SE efficacy". That is, the method for calculating the self-efficacy SE obtained from the measurement data is changed in stages. The stages in which the calculation method is changed are not limited, obviously, to two stages. The method may be changed, gradually, in three stages or more. In view of the above, the self-efficacy SE with respect to the goal biological change of the subject person can be expressed the following equation$SE = f \left({SE}_{D},{SE}_{E},{SE}_{S},…\right) .$ where SE_{D} represents SE for meals, SEE represents SE for exercises, and SEs represents SE for sleep. The function f is set optionally according to the target self-efficacy. Also, each SE is constituted by the efficacy expectation "SE efficacy" as the antecedent factor and the outcome expectation "SE outcome" as the result factor, and can be expressed by the corresponding equation as stated below. Note that the content of the function f actually differs from one another (which is also applied to the description below).$SED = f \left(SE efficacy, SE outcome\right)$ $SEE = f \left(SE efficacy, SE outcome\right)$ $SES = f \left(SE efficacy, SE outcome\right) .$

FIG. 11 is a table showing examples of elements constituting the SE efficacy and the SE outcome, and their correspondence relations with the meal SED and the exercise SEE. FIG. 12 is a table showing index examples corresponding to a small classification of the constituent elements in FIG. 11.

As to the meal SED, the items marked by sign "○" in the column SE_{D} in FIG. 11 are deemed as SE constituent elements. The efficacy expectation "SE efficacy" is calculated based on: a meal control W_{ME} that represents the subject person's expectation that he/she is to succeed at improvement of eating habits for losing weight; and measurement acts F and T. The outcome expectation "SE outcome" is calculated based on: a biological change amount ΔW that represents the subject person's expectation that such a behavior is to lead to loss in weight; and a goal G. Other than the above, it is possible to consider the subject person's environments (the number of execution of the program, elapsed days, working form, profession, job type and the like) and the subject person's attributes (sex, age and the like), or to change the evaluation expression. Also, it is possible to change the evaluation target so as to be suitable for the subject person by constantly monitoring the change in the measurement activity, or to add a barrier SE. In this way, the accuracy is improved. The barrier SE is an external environment information such as the weather, and when the number of steps taken is constant regardless of the weather, it can be evaluated that the subject person has a high barrier SE.

Here, the meal control W_{ME} is classified into an appropriate amount of meal (will to suppress the amount of meal to the appropriate level), variations in the amount of meal (will to take a constant amount of meal every day), excessive diet (will not to execute the excessive diet) and the like.

The measurement acts F and T are classified into the measurement number F (interest in the bodily changes), the measurement time T (will to stably maintain the life rhythm) and the like.

The biological change amount ΔW is classified into the weight change (degree of realization that the work to improve the lifestyle habits leads to the results, i.e., the weight value), the body composition change (degree of realization that the work to improve the lifestyle habits leads to the results, i.e., the body composition such as the visceral fat) and the like.

The goal G means a goal value (degree of the subject person's vision how much he/she can control the weight through the work to improve the lifestyle habits) and the like. However, the above are merely shown as examples. For example, the subject person can be evaluated to have a high meal SED when he/she has: the meal control WME almost constant throughout one week; the high measurement acts F and T; the biological change amount ΔW as decrease in the weight; and the high goal G. Also, as shown in FIG. 12, the difference between the weight of the morning and the weight of the evening (weight difference between morning and evening) per week is used for the meal control WME. The period is not limited to one week. It may also be one month, one year, a specific day of the week, a season or the like. Also, in place of the weight difference between morning and evening, it is possible to use the weight difference from the evening to the morning, the weight difference from the morning to the next morning, the weight difference from the evening to the next evening or the like. Furthermore, it is possible to calculate the self-efficacy for meals by estimating the calorie intake based on photos of meals.

As to the exercise SEE, similarly to the above, the items marked by sign "○" in the column SEE in FIG. 11 are deemed as SE constituent elements. The efficacy expectation "SE efficacy" is calculated based on the measurement acts F and T, and an activity S. The outcome expectation "SE outcome" is calculated based on the biological change amount ΔW and the goal value G.

Here, the measurement acts F and T, the biological change amount ΔW and the goal G are the same as those described above. The activity S is classified into the amount of activity (will for exercise habits, i.e., will to walk), the change in the amount of activity (will to increase the amount of activity compared to the beginning) and the like. However, the above are also merely shown as examples.

For example, the number of steps taken for one week is used for the activity S. The period is not limited to one week. It may also be one month, one year, a specific day of the week, a season or the like. The index is not limited to the number of steps taken. Examples of the index includes the calorie consumption, the calorie for activity, the fat consumption, the metabolic equivalents (METs), the exercises (Ex), the walking distance, the sitting time or the like. However, it is not limited thereto.

As described above, each calculation expression of the meal SE_{D} and the exercise SE_{E} can be defined based on the efficacy expectation "SE efficacy" related to the lifestyle habits and the outcome expectation "SE outcome" related to the living body. That is, as to the meal SED, the SE_{D} efficacy as the efficacy expectation and the SE_{D} outcome as the outcome expectation can be expressed respectively by the following equations${SE}_{D} efficacy = f \left({W}_{ME}, F, T\right)$ ${SE}_{D} outcome = f \left(ΔW, G\right) .$

On the other hand, as to the exercise SEE, the SEE efficacy as the efficacy expectation and the SEE outcome as the outcome expectation can be expressed respectively by the following equations$SEE efficacy = f \left(F, T, S\right)$ ${SE}_{E} outcome = f \left(ΔW, G\right) .$

FIG. 13 is a flowchart exemplarily showing a method for formulating respective calculation expressions for the meal SE_{D} and the exercise SE_{E} by the multiple regression analysis. FIGS. 14 are graphs showing respectively correlations between the meal SE_{D}/exercise SEE obtained by the method indicated in FIG. 13 and the self-efficacy based on the questionnaire. Since the multiple regression analysis is a publicly known method, the detail description thereon is omitted.

In order to avoid the overfitting and the multicollinearity, the quantitative evaluation is performed using the statistical criteria, specifically, the Akaike information criterion (AIC) and the variance inflation factor (VIF), so as to select variables.

Specifically, as shown in FIG. 13, unavailable data is removed from the evaluation object data (in this example, the number of samples N = 127) by maintaining only the all explanatory variables possession data (in this example, N = 67).

Next, one variable is selected from each SE category so as to create variable sets by round robin processing (s11). In this example, approximately 910 thousand sets were created as the variable sets for the meal SED, and approximately 360 thousand sets were created as the variable sets for the exercise SE_{E}.

Then, the multiple regression analysis is performed so as to calculate the AIC and the VIF (s12). For example, the explanatory variable is determined (s13) so that the AIC is minimum under the condition of $Maximum value of VIF < 5 ,$ and after evaluating the accuracy (s14), the correlation coefficient is calculated (s15).

As the questionnaire as to the self-efficacy for meals, the questions (full marks: 24 points) are used, which are described in Satoshi, Shimai et al., "Development of Self-efficacy Measures for Eating Behavior - Validity and Reliability of Japanese Version of Situational Appetite Measure (KC-SAM) and Diet Efficacy Measure (KC-DEM)-." Kobe Jogakuin Daigaku Ronsbu (Kobe College Journal), volume 47(1), 2000, pp.129-139. As the questionnaire as to the self-efficacy for exercises, the questions (full marks: 25 points) are used, which are described in Koichiro, Oka. "Stages of Change for Exercise Behavior and Self-efficacy for Exercise among Middle-Aged Adults." Japanese Society of Public Health, volume 50, no.3, 2003, pp. 208-215. As shown in FIG. 13, the calculated correlation coefficient was R = 0.60 for the meal SE_{D} and R = 0.63 for the exercise SEE.

As an example of the presentation of the evaluation points, it is possible to display the evaluation points as a radar chart as shown in FIGS. 15 and 16(a). In this way, it is easy to recognize the balance of the self-efficacies related to the various lifestyle habits. If the evaluation points are displayed as the values or levels as shown in FIG. 16(b), it is easy to have an improvement desire. If the time series variation is displayed as a graph as shown in FIG. 16(c), it is easy to recognize the difference from the other SEs and each change. Furthermore, if the time series variation is displayed as a graph with a start time as a reference as shown in FIG. 16(d), it is easy to recognize the change from the start time. However, the display manner of the evaluation points is not limited to the above examples.

Also, since the meal SE_{D} and the exercise SE_{E} can be respectively estimated with a relatively high accuracy, it is possible to display the evaluation points dividing into the efficacy expectation and the outcome expectation as a radar chart, as shown in FIG. 17.

The lack of the measurement information sometimes means that the subject person could not perform the measurement against his/her will because of an extraordinary action such as a business trip or a trip. As to the activity monitor 3A, the subject person may sometimes forget to carry it. In these cases, it is not preferable to simply determine that the subject person neglected the measurement.

In view of the above, the reason for the lack of the measurement information may be estimated based on, for example, the past measurement information and the like. Then, when there is a low possibility that the subject person neglected the measurement, the lacked value may be interpolated based on the past measurement information so as to eliminate the influence of the extraordinary actions. As a method for interpolating the lack, it is possible to replace the lack value with the past measurement information. Also, as a value for interpolating the lack, it is possible to use past similar information measured, for example, on weekdays, on weekends, on a certain day of the week, or at a certain time, in accordance with the subject person's lifestyle. Furthermore, it is possible to estimate the subject person's action when there is lack of the measurement information further in consideration of the subject person's behavior information (for example, GPS information) recorded in the portable terminal 2.

### <Other Embodiments>

In the above-described embodiments, it is possible to use different evaluation expressions of the self-efficacy according to, for example, the measurement frequency, the elapsed days for the program, the attributes or the goal value.

Also, in the above-described embodiments, the psychological characteristics (for example, self-efficacy) of the subject person are evaluated based on the measurement information obtained by measuring the subject person's biological information or behavior information. However, in order to evaluate the psychological characteristics of the subject person, it is also possible to combine the above with a questionnaire-answering expression.

With the above configuration, it is possible to calculate more accurately the psychological characteristics of the subject person. Also, compared to the conventional method for estimating the self-efficacy and the like from only the results of the questionnaire, the amount of each questionnaire can be reduced. Thus, it is possible to reduce the subject person's burden in answering the questions, which leads to reduction in biased answers and in great variations in answers. Furthermore, since the temporal change can be confirmed, it is possible to calculate more accurately the psychological characteristics of the subject person.

This application claims priority on Patent Application No. 2014-210865 filed in Japan on October 15, 2014 and Patent Application No. 2015-165203 filed in Japan on August 24, 2015. The entire contents thereof are hereby incorporated in this application by reference. Also, the entire contents of the documents cited herein are hereby incorporated in this application by reference.

### Description of Reference Numerals

- 1: Characteristic evaluation apparatus
- 2: Portable terminal
- 2A: Portable terminal
- 3: Weight scale
- 3x: Weight scale
- 3A: Activity monitor
- 3a: Activity monitor functions
- 11: Arithmetic processing unit
- 12: Subject person database (subject person DB)
- 13: Measurement history database (measurement history DB)
- 14: Communication unit

## Claims

1. A characteristic evaluation apparatus, comprising:
an input unit configured o input: measurement information obtained by measuring biological information or behavior information, attribute information, or goal information of a subject person;
an information storage unit configured to store the measurement information, the attribute information, or the goal information input from the input unit, and
an evaluation unit configured to evaluate psychological characteristics of the subject person with respect to a biological change or a behavioral change as a goal for the subject person based on the measurement information, the attribute information, or the goal information.

2. The characteristic evaluation apparatus according to claim 1,
wherein the measurement information that is stored in the information storage unit includes at least a measurement date and time, and
wherein the evaluation unit evaluates the psychological characteristics by means of evaluation points according to a measurement condition that is obtained from a history of the measurement information.

3. The characteristic evaluation apparatus according to claim 2,
wherein the evaluation unit changes, in stages, a method for calculating the evaluation points calculated based on the measurement information when a measurement frequency included in the measurement condition, the measurement information, the attribute information, or the goal information are not less than respectively predetermined threshold values, or meet respectively predetermined conditions.

4. The characteristic evaluation apparatus according to claim 2 or 3,
wherein the evaluation unit interpolates lack of the measurement information based on the behavior information.

5. The characteristic evaluation apparatus according to any one of claims 2 to 4, further comprising an output unit configured to output evaluation results evaluated by the evaluation unit with respect to the psychological characteristics of the subject person,
wherein the output unit outputs, to the subject person, a message as the evaluation results according to the evaluation points evaluated by the evaluation unit with respect to the psychological characteristics of the subject person.

6. The characteristic evaluation apparatus according to claim 5,
wherein the measurement information stored in the information storage unit includes a measurement value associated with the measurement date and time when the biological information or the behavior information is measured, and
wherein the output unit outputs, to the subject person, a message as the evaluation results according to: the evaluation points evaluated by the evaluation unit with respect to the psychological characteristics of the subject person; and a change in the measurement value.

7. A characteristic evaluation system comprising:
a measurement instrument configured to measure biological information or behavior information of a subject person; and
the characteristic evaluation apparatus according to any one of claims 1 to 6,
wherein the measurement information measured by the measurement instrument, the attribute information, or the goal information are directly or indirectly transmitted to the input unit of the characteristic evaluation apparatus.

8. A characteristic evaluation method comprising:
an information storage step of storing: measurement information obtained by measuring biological information or behavior information; attribute information, or goal information of a subject person;
an evaluation step of evaluating psychological characteristics of the subject person with respect to a biological change or a behavioral change as a goal for the subject person based on the measurement information, the attribute information, or the goal information of the subject person that are stored in the information storage step; and
an output step of outputting evaluation results evaluated with respect to the psychological characteristics of the subject person in the evaluation step.

9. A characteristic evaluation program configured to cause a computer to execute the characteristic evaluation method according to claim 8.
